(19) Europäisches Patentamt
European Patent Office
Office européen des brevets

(11) **EP 1 877 387 B1**

(12) **EUROPÄISCHE PATENTSCHRIFT**

(45) Veröffentlichungstag und Bekanntmachung des Hinweises auf die Patenterteilung:
**06.10.2010 Patentblatt 2010/40**

(21) Anmeldenummer: **06742871.4**

(22) Anmeldetag: **03.05.2006**

(51) Int Cl.:
**C07D 251/62** *(2006.01)*

(86) Internationale Anmeldenummer:
**PCT/EP2006/004410**

(87) Internationale Veröffentlichungsnummer:
**WO 2006/117243 (09.11.2006 Gazette 2006/45)**

(54) **VERFAHREN UND VORRICHTUNG ZUR KRISTALLISATION VON MELAMIN**

METHOD AND DEVICE FOR CRYSTALLIZING MELAMINE

PROCEDE ET DISPOSITIF DE CRISTALLISATION DE LA MÉLAMINE

(84) Benannte Vertragsstaaten:
**AT CH DE IT LI NL PL**

(30) Priorität: **04.05.2005 DE 102005021802**

(43) Veröffentlichungstag der Anmeldung:
**16.01.2008 Patentblatt 2008/03**

(73) Patentinhaber: **AMI Agrolinz Melamine International GmbH**
**4021 Linz (AT)**

(72) Erfinder:
• **RUECH, Wolfgang**
**A-4753 Taiskirchen (AT)**
• **NEUMÜLLER, Christoph**
**A-4482 Ennsdorf (AT)**
• **WALLEK, Thomas**
**A-4400 Steyr (AT)**

(74) Vertreter: **Gross, Felix et al**
**Patentanwälte Maikowski & Ninnemann**
**Postfach 15 09 20**
**10671 Berlin (DE)**

(56) Entgegenhaltungen:
WO-A-01/46159      WO-A-95/06042
WO-A-03/022823     US-A- 3 637 686

## Beschreibung

Die Anmeldung betrifft ein Verfahren zur zweistufigen Kristallisation von Melamin gemäß Anspruch 1 sowie eine Vorrichtung gemäß Anspruch 27.

Melamin wird industriell nahezu ausschließlich aus Harnstoff nach folgender Reaktionsgleichung hergestellt:

$$6\ H_2N\text{-}CO\text{-}NH_2 \rightarrow C_3N_3(NH_2)_3 + 6\ NH_3 + 3\ CO_2$$

Die Melaminherstellverfahren können in zwei Kategorien unterteilt werden. Es gibt nichtkatalytische Hochdruckverfahren, bei welchen das Melamin bei Drücken größer 70 bar und Temperaturen größer 330 °C in flüssiger Phase hergestellt wird. Bei den katalytischen Niederdruckverfahren erfolgt, ebenfalls ausgehend von Harnstoff, die Melaminsynthese in der Gasphase.

Allen Melaminverfahren ist gemeinsam, dass das rohe Melamin aus der Synthesestufe anschließend gereinigt werden muss, da es unter anderem schwerlösliche Kondensationsnebenprodukte wie zum Beispiel Melem und Melam enthält.

Zum Abbau dieser Nebenprodukte wird das rohe Melamin meist in Gegenwart von Wasser aufgearbeitet, da auf diese Weise die Kondensationsnebenprodukte zu Oxoaminotriazinen (OAT) hydrolisiert werden. Letztere sind im alkalischen Bereich im Gegensatz zum Melamin löslich, so dass Melamin aus der alkalischen wässrigen Lösung auskristallisiert werden kann, während sich die OAT in gelöster Form in der Kristallisationsmutterlauge befinden.

Zusätzlich zu den OAT enthält die Kristallisationsmutterlauge eine gewisse Menge an Melamin, welche der Melaminlöslichkeit bei der im Kristallisator herrschenden Temperatur entspricht. Diese Löslichkeit beträgt beispielsweise bei 40 °C noch etwa 0,72 Gew. % Melamin. Die Kristallisationsmutterlauge und damit auch das darin enthaltene Melamin wird großteils als Abwasser aus der Melaminanlage abgeführt und beispielsweise in einer thermischen Abwasseraufarbeitungsanlage (TAA) behandelt, wo die Abwasserinhaltsstoffe in $CO_2$ und $NH_3$ zersetzt werden. Dieses Verfahren ist beispielsweise in der italienischen Patentschrift 01282370 beschrieben. Hier wird eine 0,85 Gew-% melamin- und 0,40 Gew-% OAT hältige Mutterlauge aus der Melaminkristallisation im geschlossenen Gefäß auf 180 bis 250 °C erhitzt und für 20 bis 120 min belassen, wodurch Melamin und OAT abgebaut werden. In einem solchen Verfahren geht das in der Kristallisationsmutterlauge enthaltene Melamin nicht nur als Produktverlust in die Gesamtbilanz der Melaminanlage ein, sondern es muss darüber hinaus in der TAA thermische Energie aufgewandt werden, um das Melamin zu zerstören.

Es besteht deshalb das Bestreben, das in der Kristallisationsmutterlauge einer Melaminanlage vorhandene Melamin zu minimieren.

Gemäß WO 99/37628 A1 wird ein aus einer Melaminanlage stammendes Abwasser, welches unter anderem Melamin, Ammelin und Ammelid enthält, mit einem schwach sauren Kationenaustauscher in Kontakt gebracht. Dadurch werden die OAT Ammelin und Ammelid absorbiert, während das Melamin im Eluat gelöst ist. In einem zweiten Verfahrensschritt werden anschließend die OAT mittels einer starken Säure eluiert. Die Methode ist prinzipiell für die Abtrennung von Melamin aus Mutterlaugen anwendbar, sie ist jedoch technisch aufwendig und erfordert Regenerationszyklen für den Ionentauscher, so dass in Standby-Module investiert werden muss, auf welche periodisch umgeschaltet werden kann.

Ein Verfahren, welches eine Melaminkristallisation beschreibt, ist der US-A 3,637,686 zu entnehmen. Hier wird Melaminschmelze aus einem Hochdruckverfahren durch Kontakt mit kaltem Ammoniak verfestigt und anschließend mit einer wässrigen ammoniakhältigen Lösung bei ca. 5-100 bar zum Abbau der Nebenprodukte behandelt. Anschließend wird aus der wässrigen ammoniak- und OAT hältigen Melaminlösung durch eine zweistufige Kristallisation das Melamin auskristallisiert. Die erste Kristallisation erfolgt durch die Entspannung der Melaminlösung bei 60 bis 130 °C auf einen Druck von ca. 1-15 bar in einem Entgasungstank. Dabei wird ein geringer Teil des Melamins auskristallisiert. Im zweiten Kristallisationsschritt erfolgt die Kristallisation der Hauptmenge des Melamins bei einem Druck von 30 bis 760 mm Hg absolut und einer Temperatur von 20 bis 60 °C. Nur im Grenzfall ist damit der Atmosphärendruck gemeint, im Wesentlichen erfolgt die Kristallisation im Vakuum (d.h. bei einem Druck unterhalb des Atmosphärendrucks). Diese technische Bedeutung des Begriffs Vakuum wird im Folgenden verwendet.

Das Vakuum wird über einen Dampfejektor hergestellt. Die anfallende Kristallisationsmutterlauge, welche entsprechend der Temperatur im Vakuumkristallisator mehr oder weniger Melamin enthält, wird zum einen Teil in die Melaminanlage rückgeführt und zum anderen Teil ausgeschleust. Über den ausgeschleusten Mutterlaugenanteil kommt es zu einem Melaminverlust. Um diesen Verlust zu minimieren, muss der Vakuumkristallisator bei möglichst niedriger Temperatur das heißt möglichst hohem Vakuum betrieben werden. Dazu ist ein immens hoher Dampfeinsatz am Dampfejektor nötig, da der gesamte Kristallisatorinhalt auf diese Bedingungen gebracht werden muss.

Ein weiteres Verfahren zur Behandlung von Kristallisationsmutterlaugen eines Melaminverfahrens beschreibt die WO 01/46159 A2. Demnach wird das Melamin aus der 160 bis 170 °C heißen wässrigen Lösung mittels Kühlung auf 40 bis 50 °C bei Atmosphärendruck und einem pH größer 11 auskristallisiert. Die nach der Melaminabtrennung erhaltene melamin- und OAT hältige Mutterlauge wird angesäuert, wobei die OAT auskristallisieren. Die so erhaltene OAT-Suspension wird einer Tangentialfiltration unterzogen, in welcher melaminreiches Permeat und als Retentat eine OAT-Suspension erhalten wird. Abhängig vom Feststoffgehalt der OAT-Suspension ist dieses Verfahren mit einem unterschiedlich hohen Melaminverlust verbunden. Nachteilig dabei ist weiters die Störanfälligkeit der Filtrationseinheit, welche zu Reinigungszwecken periodisch gewechselt werden muss.

Es stellte sich demnach die Aufgabe, ein Verfahren zur Kristallisation von Melamin zu finden, welches es ermöglicht, bei vernünftigem Energieeinsatz und einfacher praktischer Betriebsweise, den Melaminverlust über die Kristallisationsmutterlauge zu minimieren.

Gegenstand der vorliegenden Erfindung ist ein Verfahren zur zweistufigen Kristallisation von Melamin, das dadurch gekennzeichnet ist, dass eine OAT-hältige wässrige Melaminlösung

a) einer ersten Kristallisationsstufe unter Vakuum bei einem pH-Wert größer 11 und einer Temperatur $T_{K1}$ unterzogen wird, wobei ein Großteil des Melamins kristallisiert, welcher der weiteren Aufarbeitung zugeführt wird und wobei eine erste OAT- und melaminhältige Mutterlauge erhalten wird, anschließend

b) mindestens ein Teil der ersten OAT- und melaminhältigen Mutterlauge mindestens einer zweiten Kristallisationsstufe unter Vakuum bei einem pH-Wert größer 11 und einer Temperatur $T_{K2}$ mit $T_{K2} < T_{K1}$ unterzogen werden, wobei ein weiterer Teil des Melamins kristallisiert, welcher in den Melaminprozess rückgeführt wird und wobei eine zweite OAT-hältige Mutterlauge erhalten wird, welche einer Abwasseraufarbeitungsstufe zugeführt wird.

Erfindungsgemäß wird aus der OAT-hältigen wässrigen Melaminlösung in einer ersten Vakuumkristallisation bei einer höheren Temperatur ein Großteil des Melamins auskristallisiert und der weiteren Aufarbeitung zugeführt. In mindestens einer zweiten Vakuumkristallisation, die bei niedrigerer Temperatur betrieben wird, wird mindestens ein Teil der in der ersten Vakuumkristallisation anfallenden melaminhältigen Mutterlauge zugeführt und darin enthaltenes Melamin auskristallisiert. Da der Melamingehalt der Mutterlauge der Löslichkeit des Melamins bei der im Kristallisator der ersten Kristallisationsstufe herrschenden Temperatur entspricht und die Löslichkeit mit abnehmender Temperatur sinkt, kann durch mindestens eine zweite Kristallisation bei niedrigerer Temperatur das restliche Melamin nahezu vollständig aus der Mutterlauge abgetrennt werden. Somit wird in der mindestens einen zweiten Kristallisationsstufe eine nahezu melaminfreie, dafür an OAT angereicherte Mutterlauge erhalten.

Ein Vorteil des erfindungsgemäßen Verfahren liegt darin, dass in der mindestens einen zweiten Kristallisationsstufe nicht der gesamte Kristallisatorinhalt der ersten Kristallisationsstufe auf die niedrige Temperatur gekühlt werden muss sondern nur jener Mutterlaugenanteil, der letztlich in die Abwasseraufarbeitungsstufe ausgeschleust wird.

Ein weiterer Vorteil des Verfahrens ist, dass durch die Abtrennung und nahezu vollständige Rückführung des Melamins aus der ersten Mutterlauge bei effizientem Energieeinsatz die Harnstoffeinsatzzahl des Gesamtprozesses gegenüber den bekannten Verfahren gesteigert werden kann.

Darüber hinaus wird durch das erfindungsgemäße Verfahren eine Energieersparnis in der der Kristallisation üblicherweise nachgeschalteten thermischen Abwasseraufarbeitungsanlage erreicht, da dort nahezu ausschließlich OAT, jedoch kein Melamin, abgebaut werden muss.

Unerwarteterweise wird beim erfindungsgemäßen Verfahren in der mindestens einen zweiten Kristallisationsstufe bei der niedrigeren Temperatur eine ebenso selektive Melaminkristallisation wie in der ersten Kristallisationsstufe, welche bei höherer Temperatur abläuft, erreicht. Dies ist überraschend, da der Fachmann erwarten würde, dass die Löslichkeit der OAT mit abnehmender Temperatur analog zur Löslichkeit des Melamins sinken würde und demnach aus der Mutterlauge gleichzeitig Melamin und OAT auskristallisierten würden.

Die OAT-hältige wässrige Melaminlösung kann aus jeder beliebigen Melaminanlage stammen. Bevorzugterweise stammt sie aus einem Hochdruckverfahren zur Herstellung von Melamin aus Harnstoff, welches einen wässrigen Aufarbeitungsteil aufweist. In einem solchen Hochdruckverfahren wird eine Rohmelaminschmelze in einem Hochdruckreaktor hergestellt und die Schmelze anschließend, gegebenenfalls nach einer oder mehrerer Zwischenstufen, in einem Quencher mit einer kalten wässrigen Lösung kontaktiert und dadurch in eine Melaminlösung oder Melaminsuspension überführt. Anschließend wird die Melaminlösung oder -suspension in Gegenwart von Wasser und gegebenenfalls Alkali aufgearbeitet, beispielsweise werden die Nebenprodukte abgebaut, gegebenenfalls der enthaltene Ammoniak weitgehend entfernt und die wässrige Melaminlösung anschließend der Kristallisation zugeführt. Die melamin- und OAT-hältige wässrige Lösung kann auch aus jedem beliebigen anderen Melaminhochdruckverfahren stammen.

Vorteilhaft ist es, wenn die OAT-hältige wässrige Melaminlösung 6 bis 15 Gew-%, bevorzugt 8 bis 12 Gew-% Melamin enthält.

Weiterhin ist es bevorzugt wenn die melamin- und OAT-hältige wässrige Lösung eine Temperatur von 100 bis 200 °C, bevorzugt von 130 bis 150 °C aufweist.

Bei diesen Bedingungen im wässrigen Aufarbeitungsteil der Melaminanlage, welcher der Kristallisation vorgeschaltet ist, können die Nebenprodukte mit ausreichender Reaktionsgeschwindigkeit abgebaut werden, während der Melaminverlust durch die gleichzeitig ablaufende Melaminhydrolyse in vertretbaren Ausmaßen gehalten wird.

Vorteilhafterweise weist die OAT-hältige wässrige Melaminlösung einen pH Wert von größer 11 auf, wobei der pH Wert über die Zugabe von alkalischen Stoffen, meist NaOH, eingestellt wird. Es ist auch möglich, direkt vor oder bei der ersten Kristallisationsstufe der wässrigen Lösung Alkali bis zum gewünschten pH Wert zuzudosieren. Ammoniak kann in der OAT-hältigen wässrigen Melaminlösung ebenfalls in geringen Mengen enthalten sein.

Besonders vorteilhaft ist es, wenn der pH-Wert in der ersten und in der mindestens einen zweiten Kristallisationsstufe 11, 8 bis 12,5 beträgt. Bei diesen pH Werten wird auch bei niedrigen Temperaturen eine sehr selektive Melaminkristallisation erreicht, während die OAT in Lösung gehalten werden.

In der ersten Kristallisationsstufe wird ein Großteil des Melamins aus der wässrigen Lösung auskristallisiert, wobei unter einem Großteil mehr als 95 Gew-% des insgesamt auskristallisierten Melamins verstanden werden. Üblicherweise werden in der ersten Kristallisationsstufe bereits bis zu 98 bis 99 Gew-% des Melamins auskristallisiert.

Es ist von Vorteil wenn die Temperatur $T_{K1}$ in der ersten Kristallisationsstufe 30 bis 60 °C, besonders bevorzugt 40 bis 50 °C beträgt. Da die erzielbare Temperatur unter anderem von der Temperatur des zur Verfügung stehenden Kühlmediums abhängt, kann die Temperatur im ersten Kristallisator je nach den vorhandenen Standortbedingungen gewählt werden. Dabei ist zu bemerken, dass zwar hinsichtlich eines geringen Melamingehaltes in der Mutterlauge eine möglichst niedrige Temperatur anzustreben ist, andererseits aufgrund der großen zu kühlenden Menge, nämlich des gesamten Kristallisatorinhaltes, bei niedriger Temperatur ein entsprechend hoher Energieeinsatz erforderlich ist. 40 bis 50°C ist diesbezüglich eine vorteilhafte Temperatur in der ersten Kristallisationsstufe.

In der ersten Kristallisationsstufe wird eine erste OAT- und melaminhältige Mutterlauge erhalten. Bevorzugt werden mindestens 20 Gew-%, insbesondere 20 bis 40 Gew-%, besonders bevorzugt etwa 30 Gew-% dieser ersten OAT- und melaminhältigen Mutterlauge mindestens einer zweiten Kristallisationsstufe unterzogen.

Dabei ist es bevorzugt, dass die erste OAT- und melaminhältige Mutterlauge, die der mindestens einen zweiten Kristallisationsstufe zugeführt wird, Mutterlauge aus der Melaminfiltration und aus der ersten Kristallisationsstufe enthält. Die Melaminfiltration ist üblicherweise der ersten Kristallisationsstufe nachgeschaltet. Dort wird der Feststoffgehalt der in der ersten Kristallisationsstufe erhaltenen etwa 10 bis 20 Gew-% igen Melaminsuspension weiter erhöht. In der Melaminfiltration fällt eine Mutterlauge an, welche hinsichtlich ihres OAT- und Melamingehaltes üblicherweise identisch mit der ersten OAT- und melaminhältigen Mutterlauge aus der ersten Kristallisationsstufe ist.

Bevorzugt ist weiters, wenn die Temperatur $T_{K2}$ in der mindestens einen zweiten Kristallisationsstufe 15 bis 40°C, besonders bevorzugt 25 bis 35 °C beträgt. Da in der mindestens einen zweiten Kristallisationsstufe die abzukühlende Menge geringer als in der ersten Kristallisationsstufe und damit der Energieeinsatz begrenzt ist, ist in der mindestens einen zweiten Kristallisationsstufe auf jeden Fall eine möglichst niedrige Temperatur anzustreben. Je niedriger die Temperatur in der zweiten Kristallisationsstufe ist, desto vollständiger kann das Melamin aus der Mutterlauge zurückgewonnen werden.

Vorteilhaft ist es, wenn die der mindestens einen zweiten Kristallisationsstufe zugeführte erste OAT- und melaminhältige Mutterlauge eine Konzentration von 1,5 - 2,5 Gew-% OAT, insbesondere bevorzugt von 1,9 bis 2,2 Gew-% OAT aufweist.

Weiters ist es vorteilhaft, wenn die in der mindestens einen zweiten Kristallisationsstufe erhaltene OAT-hältige Mutterlauge einer thermischen Abwasseraufarbeitungsanlage TAA zugeführt wird. Dort erfolgt der thermische Abbau der Abwasserinhaltsstoffe, wobei praktisch reines Wasser sowie $NH_3$ und $CO_2$, meist in wässriger Lösung, erhalten werden. Während das Wasser ausgeschleust wird, können $NH_3$ und $CO_2$ in die Melamin- oder auch Harnstoffanlage rezykliert werden. Die Verarbeitung hoch OAT belasteter Abwässer in der mindestens einen zweiten Kristallisationsstufe hat für die TAA den Vorteil, dass sie ausschließlich hochkonzentriertes OAT-hältiges Abwasser zu verarbeiten hat und damit ihre hydraulische Last vermindert wird.

Alternativ zur TAA können aus der OAT-hältigen Mutterlauge der mindestens einen zweiten Kristallisationsstufe auch die OAT abgetrennt werden.

Bevorzugt ist weiters eine Ausführungsform, bei welcher der nicht in die mindestens eine zweite Kristallisationsstufe geführte Teil der ersten OAT- und melaminhältigen Mutterlauge in den wässrigen Aufarbeitungsteil der Melaminanlage rückgeführt wird. Auf diese Weise können sowohl Frischwasser als auch Alkali im wässrigen Aufarbeitungsteil eingespart werden.

In einer vorteilhaften Ausführungsform wird der in der mindestens einen zweiten Kristallisationsstufe kristallisierte Teil des Melamins in den Quencher und/oder in die erste Kristallisationsstufe und/oder in den Suspensionsbehälter nach der ersten Kristallisationsstufe rückgeführt. Generell kann das in der mindestens einen zweiten Kristallisationsstufe kristallisierte Melamin in jeden beliebigen Teil der Melaminanlage rückgeführt werden. Durch die Tatsache, dass es nicht gemeinsam mit den OATs in die TAA gelangt, wird die Harnstoffeinsatzzahl des Gesamtprozesses auf jeden Fall verbessert und trägt somit direkt zur Melamin Ausbeuteerhöhung bei.

Generell wird in den Kristallisationsstufen die gewünschte Betriebstemperatur eingestellt und der Unterdruck ergibt sich aus dem Dampfdruck der Flüssigkeit im Kristallisator. Im Unterschied zur Kristallisation bei Normaldruck, welche durch einfache Temperaturerniedrigung mittels Wärmetauscher erfolgt und bei welcher häufig Verblockungen im Wärmetauscher auftreten und Reinigungszyklen im Kristallisator nötig sind, kommt es bei der Vakuumkristallisation zu einer Verdunstungskühlung an der Oberfläche des zu kristallisierenden Mediums. Verblockungen treten weniger häufig auf, auch weil die Vakuumkristallisatoren üblicherweise einen Umwälzkreislauf aufweisen, so dass der Kristallisatorinhalt ständig in Bewegung ist und dadurch Anbackungen am Boden verhindert werden.

Für die Herstellung des Vakuums in der ersten Kristallisationsstufe wird bevorzugterweise ein Kondensator mit Kühlmedium verwendet. Der Kondensator dient der Absaugung und Kondensation der Brüden. Aufgrund der großen abzusaugenden Brüdenmenge in der ersten Kristallisationsstufe ist diese Variante der Evakuierung am energieeffizientesten. Als Kühlmedium dient üblicherweise Kühlwasser, prinzipiell ist aber auch jedes andere Kühlmedium, das mit entsprechend niedriger Temperatur zur Verfügung steht, einsetzbar.

Für die Absaugung nicht kondensierbarer Inerte können Dampfejektoren oder Vakuumpumpen eingesetzt werden, wobei Dampfejektoren aufgrund ihrer geringeren Störanfälligkeit den Vakuumpumpen vorzuziehen sind.

Vorteilhafterweise sind für die totale Brüdenkondensation ein oder mehrere weitere Kondensatoren und für die Inertenabsaugung ein oder mehrere weitere Dampfejektoren nötig. Meist werden ein bis drei Kondensator-Dampfejektor-Stufen für die Totalkondensation und Inertenabsaugung eingesetzt. Die kondensierten Kristallisatorbrüden können beispielsweise in den wässrigen Aufarbeitungsteil der Melaminanlage rückgeführt werden.

Die Höhe des Vakuums ist von der Menge des Kühlmediums im Kondensator und von der Menge an Treibdampf im Dampfejektor beziehungsweise von der Leistung der Vakuumpumpe abhängig.

In einer bevorzugten Ausführungsvariante wird sowohl in der ersten als auch in der mindestens einen zweiten Kristallisationsstufe das Vakuum mittels Kondensator mit Kühlmedium hergestellt. Als Kühlmedium sowohl der ersten als auch der mindestens einen zweiten Krisallisationsstufe dient vorteilhafterweise Kühlwasser, prinzipiell ist aber auch jedes andere Kühlmedium, das mit entsprechend niedriger Temperatur zur Verfügung steht, einsetzbar. Der Vorteil dieser Variante liegt darin, dass durch die höhere Temperatur $T_{K1}$ in der ersten Kristallisationsstufe gegenüber $T_{K2}$ in der ersten Kristallisationsstufe höhertemperiertes Kühlmedium zum Einsatz kommen kann. Kühlmedium niedriger Temperatur wird nur in der mindestens einen zweiten Kristallisationsstufe benötigt, wobei hier geringere Mengen als in der ersten Kristallisationsstufe vonnöten sind. Dies bedeutet eine Kühlmedium- und damit Energieersparnis gegenüber einer Variante, bei der die gesamte Kristallisation in einer einzigen Kristallisationsstufe bei $T_{K2}$ erfolgt.

In einer weiteren bevorzugten Ausführungsform wird in der ersten Kristallisationsstufe das Vakuum mittels Kondensator mit Kühlmedium und in der mindestens einen zweiten Kristallisationsstufe mittels Dampfejektor hergestellt. Diese Ausführungsform ist dann von Vorteil, wenn standortbedingt kein ausreichend niedrig temperiertes Kühlmedium zur Verfügung steht. Es kann dann in der ersten Kristallisationsstufe Kühlmedium höherer Temperatur in großer Menge eingesetzt werden, während die niedrigere Temperatur in der mindestens einen zweiten Kristallisationsstufe über die entsprechende Menge an Treibdampf im Dampfejektor erreicht wird.

Als Treibdampf wird beispielsweise 3,5 bar Dampf verwendet. Bevorzugt beträgt das Mengenverhältnis von Treibdampf zur Menge an Verdampftem zwischen 1, 0 und 1,1. Der Treibdampf verursacht die Absaugung der gesamten Brüden der mindestens einen zweiten Kristallisationsstufe mit einem kombinierten Dampfejektor-Kondensator-Vakuumsystem.

Vorteilhaft ist weiters eine Ausführungsvariante, bei welcher die Kondensation der Brüden aus der ersten und aus der mindestens zweiten Vakuumkristallisation in einem gemeinsamen Kondensator mit Kühlmedium erfolgt. Dabei sind die Brüden der zweiten Vakuumkristallisation vorzugsweise die im Dampfejektor der mindestens einen zweiten Kristallisationsstufe erzeugten komprimierten Brüden. Diese Variante bringt eine Investitionskostenersparnis, da nur ein Brüdenkondensator für beide Kristallisationsstufen benötigt wird.

Weiters ist es bevorzugt, dass die erste Kristallisationsstufe in zwei parallel geschalteten Kristallisatoren und die mindestens eine zweite Kristallisationsstufe in einem einzigen Kristallisator durchgeführt wird. Die in den beiden ersten Kristallisationsstufen erhaltenen OAT- und melaminhältigen Mutterlaugenströme werden vor dem Eintritt in die mindestens eine zweite Kristallisationsstufe vereinigt, wobei auch hier die Mutterlaugenströme der Melaminfiltrationen beigemengt werden können.

Weitere Ausführungsvarianten des erfindungsgemäßen Verfahrens, in welchen zwei oder mehr erste und/oder die mindestens eine zweite Kristallisationsstufen installiert sind, welche parallel oder in Serie geschaltet sind, sind ebenfalls möglich.

Gegenstand der Erfindung ist auch eine Vorrichtung zur Durchführung des erfindungsgemäßen Verfahrens. Die Vorrichtung weist mindestens einen ersten Vakuumkristallisator mit

- einer Vorrichtung zur Herstellung des Vakuums und zur Brüdenkondensation,
- eine Leitung zur Zuführung der OAT-hältigen wässrigen Melaminlösung,
- einer Leitung zur Abführung des kristallisierten Melamins,
- einer Brüdenabzugsleitung,
- einer Abzugsleitung mit einer Abzugspumpe für eine erste OAT- und melaminhältige Mutterlauge und

und mindestens einen zweiten Vakuumkristallisator mit

- einer Vorrichtung zur Herstellung des Vakuums und zur Brüdenkondensation,
- eine Leitung zur Zuführung der ersten OAT- und melaminhältigen Mutterlauge
- einer Leitung zur Abführung des kristallisierten Melamins,
- einer Brüdenabzugsleitung,
- einer Abzugsleitung mit einer Abzugspumpe für eine zweite Mutterlauge

auf.

Vorteilhafte Ausführungsformen sind in den Unteransprüchen angegeben.

Das mit dem vorliegenden Kristallisationsverfahren erhältliche Melamin weist eine Reinheit von mindestens 99,8 % auf.

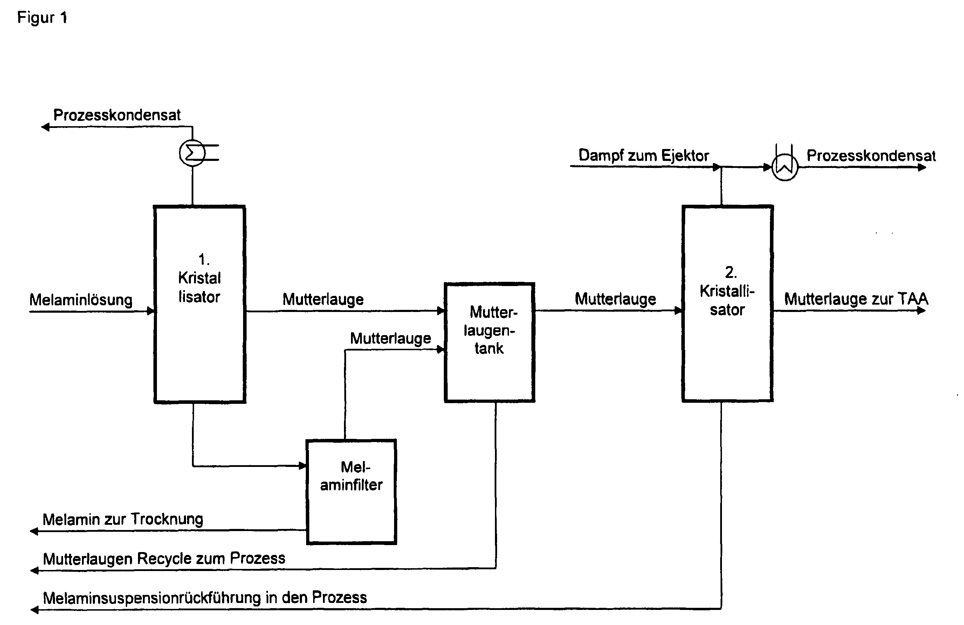

In Fig. 1 ist beispielhaft eine Ausführungsform des erfindungsgemäßen Verfahrens beschrieben.

Die OAT- hältige wässrige Melaminlösung aus der Melaminanlage wird der ersten Kristallisationsstufe (erster Kristallisator) zugeführt. Das Vakuum wird in der ersten Kristallisationsstufe mittels Kondensator mit Kühlmedium hergestellt. Die kondensierten Kristallisationsbrüden werden als Prozesskondensat in den Melaminprozess rückgeführt. Das in der ersten Kristallisationsstufe kristallisierte Melamin gelangt in Form einer Melaminsuspension zur Melaminfiltration, das dort erhaltene Melamin wird anschließend zur Trocknung geführt. Die Mutterlauge der Melaminfiltration wird gemeinsam mit der ersten OAT- und melaminhältigen Mutterlauge einem Mutterlaugentank zugeführt, in welchem die beiden Ströme vermengt werden. Ein Teil der Mutterlauge aus dem Mutterlaugentank wird in den Melaminprozess rezykliert, ein weiterer Teil gelangt in die zweite Kristallisationsstufe (zweiter Kristallisator). In dieser Kristallisationsstufe wird das Vakuum mittels Dampfejektor erreicht. Für die Kondensation der Brüden ist ein Kondensator installiert, der mit einem Kühlmedium betrieben wird. Die OAT-hältige Mutterlauge aus der zweiten Kristallisationsstufe wird in eine thermische Abwasseraufarbeitungsanlage TAA geführt, während die Melaminsuspension aus der zweiten Kristallisationsstufe in den Melaminprozess rezykliert wird.

Der erste Kristallisator bildet in diesem Ausführungsbeispiel die erste Kristallisationsstufe, der zweite Kristallisator die zweite Kristallisationsstufe. Grundsätzlich ist es aber möglich, dass in einer Kristallisationsstufe mehrere Kristallisatoren parallel geschaltet sind. Auch ist es möglich, nach der zweiten Kristallisationsstufe weitere Kristallisationsstufen anzuschließen, deren Betriebsbedingungen entsprechend angepasst werden.

Im Folgenden wird die Erfindung anhand von Beispielen erläutert.

Es wird als Vergleichsvariante der Energieverbrauch eines einstufigen Kristallisationsverfahrens dem erfindungsgemäßen zweistufigen Kristallisationsverfahren gegenübergestellt. Die Berechnung ist auf die Einheit 1t / h Melamin normiert.

Vergleichsbeispiel 1 Einstufige Kristallisation auf 30°C

Bei einer Melaminkonzentration von 8% und einer Temperatur von 130°C der OAT-hältigen wässrigen Melaminlösung im Zulauf zur ersten Kristallisationsstufe und einer Kristallisationstemperatur von 30 °C muss eine Wärmemenge von

$$Q = \text{Masse}_{\text{Melaminlösung}} / 3600 \times c_p \times (T_{\text{ein}} - T_{\text{krist}}) = 12500 / 3600 \times 4,2 \times (130°C - 30°C)$$
$$= 1460 \text{ kW}$$

abgeführt werden.

Beispiel 1 Zweistufige Kristallisation auf 50°C in der ersten und 30°C in der zweiten Kristallisationsstufe:

Bei derselben OAT-hältigen wässrigen Melaminlösung wie in Vergleichsbeispiel 1 und einer Kristallisationstemperatur von 50 °C muss eine Wärmemenge von 1170 kW abgeführt werden. Für die weitere Teilabkühlung von 50 °C auf 30 °C in der zweiten Kristallisationsstufe sind bei 4 t/h Mutterlauge bezogen auf 1 t /h Melamin 90 kW nötig. Die Treibdampfmenge für die abzuführenden Brüden aus der zweiten Kristallisation entspricht der 1 bis 1,1-fache Verdampfungsmenge. Dies kommt einer Wärmemenge von 90 kW gleich.

Für die Gesamtsumme des erfindungsgemäßen Verfahren ergibt sich eine Wärmemenge von 1170 + 90 + 90 kW = 1350 kW.

Somit wird eine Energieersparnis pro t Melamin von
1460 - 1350 = 110 kW erreicht.

Beispiel 2 Verbesserung Harnstoffeinsatzkennzahl:

Es wird die in der erfindungsgemäßen zweiten Kristallisationsstufe bei 30 °C aus der Mutterlauge zurückgewonnene Menge an Melamin und die so erreichbare Verbesserung der Harnstoffeinsatzkennzahl errechnet.

Bezogen auf 4 t Abwasser pro t Melamin sind bei einer Kristallisationstemperatur von 50 °C in der ersten Kristallisationsstufe in der Mutterlauge 1,02% Melamin gelöst. Dies entspricht einer Absolutmenge von 40,8 kg Melamin in der ersten OAT- und melaminhältigen Mutterlauge bezogen auf 1 t produziertes Melamin.

Bei einer Kristallisationstemperatur von 30 °C in der zweiten Kristallisationsstufe sind in der Mutterlauge der zweiten Kristallisationsstufe 0,48 % Melamin gelöst, entsprechend einer Absolutmenge von 19,2 kg bezogen auf 1 t produziertes Melamin.

Damit ergibt sich durch die zweite Kristallisationsstufe pro t produziertem Melamin eine Rückgewinnung von 21,6 kg Melamin aus der Mutterlauge der ersten Kristallisationsstufe . Dies entspricht einer Harnstoffersparnis von 65 kg / t Melamin, das heißt, der Harnstoffeinsatz verringert sich von üblicherweise 3,1 t / t Melamin auf 3,035 t / t Melamin.

**Patentansprüche**

1. Verfahren zur zweistufigen Kristallisation von Melamin
   **dadurch gekennzeichnet, dass**
   eine Oxoaminotriazin (OAT)-hältige wässrige Melaminlösung

   a) einer ersten Kristallisationsstufe unter Vakuum bei einem pH-Wert größer 11 und einer Temperatur $T_{K1}$ unterzogen wird, wobei ein Großteil des Melamins kristallisiert, welcher der weiteren Aufarbeitung zugeführt wird und wobei eine erste OAT- und melaminhältigen Mutterlauge erhalten wird, anschließend

   b) mindestens ein Teil der ersten OAT- und melaminhältigen Mutterlauge mindestens einer zweiten Kristallisationsstufe unter Vakuum bei einem pH-Wert größer 11 und einer Temperatur $T_{K2}$ mit $T_{K2} < T_{K1}$ unterzogen werden, wobei ein weiterer Teil des Melamins kristallisiert, welcher in den Melaminprozess rückgeführt wird und wobei eine zweite OAT-hältige Mutterlauge erhalten wird, welche einer Abwasseraufarbeitungsstufe zugeführt wird.

2. Verfahren nach Anspruch 1, **dadurch gekennzeichnet, dass** es an ein Hochdruckverfahren zur Herstellung von Melamin aus Harnstoff mit wässriger Aufarbeitung gekoppelt ist.

3. Verfahren nach Anspruch 1 oder 2, **dadurch gekennzeichnet, dass** die OAT-hältige wässrige Melaminlösung 6 bis 15 Gew% Melamin enthält.

4. Verfahren nach mindestens einem der vorher genannten Ansprüche, **dadurch gekennzeichnet, dass** die OAT-hältige wässrige Melaminlösung 8 bis 12 Gew-% Melamin enthält.

5. Verfahren nach mindestens einem der vorher genannten Ansprüche, **dadurch gekennzeichnet, dass** die OAT-hältige wässrige Melaminlösung eine Temperatur von 100 bis 200 °C aufweist.

6. Verfahren nach mindestens einem der vorher genannten Ansprüche, **dadurch gekennzeichnet, dass** die OAT-hältige wässrige Melaminlösung eine Temperatur von 130 bis 150 °C aufweist.

7. Verfahren nach mindestens einem der vorher genannten Ansprüche, **dadurch gekennzeichnet, dass** der pH Wert mittels NaOH eingestellt wird.

8. Verfahren nach mindestens einem der vorher genannten Ansprüche, **dadurch gekennzeichnet, dass** der pH-Wert in der ersten und mindestens einer zweiten Kristallisationsstufe 11, 8 bis 12,5 beträgt.

9. Verfahren nach mindestens einem der vorher genannten Ansprüche, **dadurch gekennzeichnet, dass** $T_{K1}$ = 30 bis 60 °C beträgt.

10. Verfahren nach mindestens einem der vorher genannten Ansprüche, **dadurch gekennzeichnet, dass** $T_{K1}$ = 40 bis 50 °C beträgt.

11. Verfahren nach mindestens einem der vorher genannten Ansprüche, **dadurch gekennzeichnet, dass** mindestens 20 Gew-%, insbesondere 20 bis 40 Gew-% der ersten OAT- und melaminhältigen Mutterlauge mindestens einer zweiten Kristallisationsstufe unterzogen werden.

12. Verfahren nach mindestens einem der vorher genannten Ansprüche, **dadurch gekennzeichnet, dass** etwa 30 Gew-% der ersten OAT- und melaminhältigen Mutterlauge mindestens einer zweiten Kristallisationsstufe unterzogen werden.

13. Verfahren nach mindestens einem der vorher genannten Ansprüche, **dadurch gekennzeichnet, dass** die erste OAT- und melaminhältige Mutterlauge, die der mindestens einen zweiten Kristallisationsstufe zugeführt wird, Mutterlauge aus der Melaminfiltration enthält.

14. Verfahren nach mindestens einem der vorher genannten Ansprüche, **dadurch gekennzeichnet, dass** $T_{K2}$ = 15 bis 40 °C beträgt.

15. Verfahren nach mindestens einem der vorher genannten Ansprüche, **dadurch gekennzeichnet, dass** $T_{K2}$ = 25 bis 35 °C beträgt.

16. Verfahren nach mindestens einem der vorher genannten Ansprüche, **dadurch gekennzeichnet, dass** die der mindestens einen zweiten Kristallisationsstufe zugeführte erste OAT- und melaminhältige Mutterlauge eine Konzentration von 1,5 - 2,5 Gew-% OAT aufweist.

17. Verfahren nach mindestens einem der vorher genannten Ansprüche, **dadurch gekennzeichnet, dass** die der mindestens einen zweiten Kristallisationsstufe zugeführte erste OAT- und melaminhältige Mutterlauge eine Konzentration von 1,9 bis 2,2 Gew-% OAT aufweist.

18. Verfahren nach mindestens einem der vorher genannten Ansprüche, **dadurch gekennzeichnet, dass** die Abwasseraufarbeitungsstufe, welcher die zweite OAT-hältige Mutterlauge zugeführt wird, eine thermische Abwasseraufarbeitungsanlage TAA ist.

19. Verfahren nach mindestens einem der vorher genannten Ansprüche, **dadurch gekennzeichnet, dass** der nicht in die mindestens eine zweite Kristallisationsstufe geführte Teil der ersten OAT- und melaminhältigen Mutterlauge in den wässrigen Aufarbeitungsteil der Melaminanlage rückgeführt wird.

20. Verfahren nach mindestens einem der vorher genannten Ansprüche, **dadurch gekennzeichnet, dass** der in der mindestens einen zweiten Kristallisationsstufe kristallisierte Teil des Melamins in den Quencher, in die erste Kristallisationsstufe oder in den Suspensionsbehälter nach der ersten Kristallisationsstufe rückgeführt wird.

21. Verfahren nach mindestens einem der vorher genannten Ansprüche, **dadurch gekennzeichnet, dass** in der ersten Kristallisationsstufe das Vakuum mittels Kondensator mit Kühlmedium hergestellt wird.

22. Verfahren nach mindestens einem der vorher genannten Ansprüche, **dadurch gekennzeichnet, dass** in der ersten und in der mindestens einen zweiten Kristallisationsstufe das Vakuum mittels Kondensator mit Kühlmedium hergestellt wird.

23. Verfahren nach mindestens einem der vorher genannten Ansprüche, **dadurch gekennzeichnet, dass** in der ersten Kristallisationsstufe das Vakuum mittels Kondensator mit Kühlmedium und in der mindestens einen zweiten Kristallisationsstufe mittels Dampfejektor hergestellt wird.

24. Verfahren nach Anspruch 23, **dadurch gekennzeichnet, dass** beim Dampfejektor der mindestens einen zweiten Kristallisationsstufe das Mengenverhältnis von Einsatzdampf zur Menge an Verdampftem zwischen 1,0 und 1,1 beträgt.

25. Verfahren nach mindestens einem der vorher genannten Ansprüche, **dadurch gekennzeichnet, dass** die Kondensation der Brüden der ersten und der mindestens einen zweiten Kristallisationsstufe in einem gemeinsamen Kondensator mit Kühlmedium erfolgt.

26. Verfahren nach mindestens einem der vorher genannten Ansprüche, **dadurch gekennzeichnet, dass** zwei erste Kristallisationsstufen parallel und eine zweite Kristallisationsstufe betrieben werden.

**27.** Vorrichtung zur Durchführung des Verfahrens nach Anspruch 1 **gekennzeichnet durch** mindestens einen ersten Vakuumkristallisator mit

a) einer Vorrichtung zur Herstellung des Vakuums und zur Brüdenkondensation,
b) einer Leitung zur Zuführung der OAT-hältigen wässrigen Melaminlösung,
c) einer Leitung zur Abführung des kristallisierten Melamins,
d) einer Brüdenabzugsleitung,
e) einer Abzugsleitung mit einer Abzugspumpe für eine erste OAT-und melaminhältige Mutterlauge und

und mindestens einen zweiten Vakuumkristallisator mit

f) einer Vorrichtung zur Herstellung des Vakuums und zur Brüdenkondensation,
g) einer Leitung zur Zuführung der ersten OAT- und melaminhältigen Mutterlauge
h) einer Leitung zur Abführung des kristallisierten Melamins,
i) einer Brüdenabzugsleitung,
j) einer Abzugsleitung mit einer Abzugspumpe für eine zweite OAT-hältige Mutterlauge.

**28.** Vorrichtung nach Anspruch 27, **dadurch gekennzeichnet, dass** der mindestens eine erste Vakuumkristallisator eine Kreislaufleitung mit einer Umwälzpumpe und einer Einspeisestelle zur Zuführung der OAT-hältigen wässrigen Melaminlösung aufweist.

**29.** Vorrichtung nach Anspruch 27 oder 28, **dadurch gekennzeichnet, dass** der mindestens eine erste Vakuumkristallisator eine Zuleitung für eine Suspension aus mindestens einer zweiten Kristallisationsstufe aufweist.

**30.** Vorrichtrung nach mindestens einem der Ansprüche 27 bis 29, **dadurch gekennzeichnet, dass** der mindestens eine zweite Vakuumkristallisator eine Kreislaufleitung mit einer Umwälzpumpe und einer Einspeisestelle zur Zuführung einer ersten OAT- und melaminhältigen Mutterlauge aufweist.

## Claims

**1.** Process for the two-stage crystallization of melamine **characterized in that** an Oxoaminotriazines (OATs) -containing aqueous melamine solution

a) is subjected to a first crystallization stage under vacuum at a pH greater than 11 and a temperature $T_{K1}$, a majority of the melamine crystallizing which is fed to further workup, and a first OATs- and melamine-containing mother liquor being obtained, subsequently
b) at least a part of the first OATs- and melamine-containing mother liquor is subjected to at least one second crystallization stage under vacuum at a pH greater than 11 and a temperature $T_{K2}$ where $T_{K2} < T_{K1}$, a further part of the melamine crystallizing which is recycled to the melamine process, and a second OATs-containing mother liquor being obtained which is fed to a wastewater recycling stage.

**2.** Process according to Claim 1, **characterized in that** it is coupled to a high-pressure process for the production of melamine from urea having aqueous workup.

**3.** Process according to Claim 1 or 2, **characterized in that** the OATs-containing aqueous melamine solution contains 6 to 15% by weight melamine.

**4.** Process according to at least one of the preceding claims, **characterized in that** the OATs-containing aqueous melamine solution contains 8 to 12% by weight melamine.

**5.** Process according to at least one of the preceding claims, **characterized in that** the OATs-containing aqueous melamine solution has a temperature of 100 to 200°C.

**6.** Process according to at least one of the preceding claims, **characterized in that** the OATs-containing aqueous melamine solution has a temperature of 130 to 150°C.

**7.** Process according to at least one of the preceding claims, **characterized in that** the pH is set by means of NaOH.

8.  Process according to at least one of the preceding claims, **characterized in that** the pH in the first and at least one second crystallization stage is 11.8 to 12.5.

9.  Process according to at least one of the preceding claims, **characterized in that** $T_{K1}$ = 30 to 60°C.

10. Process according to at least one of the preceding claims, **characterized in that** $T_{K1}$ = 40 to 50°C.

11. Process according to at least one of the preceding claims, **characterized in that** at least 20% by weight, in particular 20 to 40% by weight, of the first OATs- and melamine-containing mother liquor are subjected to a second crystallization stage.

12. Process according to at least one of the preceding claims, **characterized in that** about 30% by weight of the first OATs- and melamine-containing mother liquor are subjected to at least one second crystallization stage.

13. Process according to at least one of the preceding claims, **characterized in that** the first OATs- and melamine-containing mother liquor which is fed to the at least one second crystallization stage contains mother liquor from the melamine filtration.

14. Process according to at least one of the preceding claims, **characterized in that** $T_{K2}$ = 15 to 40°C.

15. Process according to at least one of the preceding claims, **characterized in that** $T_{K2}$ = 25 to 35°C.

16. Process according to at least one of the preceding claims, **characterized in that** the first OATs- and melamine-containing mother liquor which is fed to the at least one second crystallization stage has a concentration of 1.5-2.5% by weight OATs.

17. Process according to at least one of the preceding claims, **characterized in that** the first OATs- and melamine-containing mother liquor which is fed to the at least one second crystallization stage has a concentration of 1.9 to 2.2% by weight OATs.

18. Process according to at least one of the preceding claims, **characterized in that** the wastewater recycling stage to which the second OATs-containing mother liquor is fed is a thermal wastewater recycling unit WRU.

19. Process according to at least one of the preceding claims, **characterized in that** the part of the first OATs- and melamine-containing mother liquor which is not fed into the at least one second crystallization stage is recirculated to the aqueous workup part of the melamine plant.

20. Process according to at least one of the preceding claims, **characterized in that** the part of the melamine which crystallized in the at least one second crystallization stage is recirculated to the quencher, to the first crystallization stage or to the suspension vessel downstream of the first crystallization stage.

21. Process according to at least one of the preceding claims, **characterized in that**, in the first crystallization stage, the vacuum is produced by means of a condenser using a cooling medium.

22. Process according to at least one of the preceding claims, **characterized in that** in the first crystallization stage and in the at least one second crystallization stage the vacuum is produced by means of a condenser using a cooling medium.

23. Process according to at least one of the preceding claims, **characterized in that**, in the first crystallization stage, the vacuum is produced by means of a condenser using a cooling medium, and in the at least one second crystallization stage, by means of a steam ejector.

24. Process according to Claim 23, **characterized in that**, in the steam ejector of the at least one second crystallization stage, the quantitative ratio of feed steam to the amount of evaporate is between 1.0 and 1.1.

25. Process according to at least one of the preceding claims, **characterized in that** the vapours of the first crystallization stage and the at least one second crystallization stage are condensed in a joint condenser using a cooling medium.

**26.** Process according to at least one of the preceding claims, **characterized in that** two first crystallization stages in parallel, and a second crystallization stage, are operated.

**27.** Apparatus for carrying out the process according to Claim 1, **characterized by** at least one **first vacuum crystallizer** having

> a) an apparatus for producing the vacuum and for vapour condensation,
> b) a line for feeding the OATs-containing aqueous melamine solution,
> c) a line for removing the crystallized melamine,
> d) a vapour take-off line,
> e) a take-off line having a take-off pump for a first OATs- and melamine-containing mother liquor and

and at least one **second vacuum crystallizer** having

> f) an apparatus for producing the vacuum and for vapour condensation,
> g) a line for feeding the first OATs- and melamine-containing mother liquor,
> h) a line for removing the crystallized melamine,
> i) a vapour take-off line,
> j) a take-off line having a take-off pump for a second OATs-containing mother liquor.

**28.** Apparatus according to Claim 27, **characterized in that** the at least one first vacuum crystallizer has a circuit line having a circulation pump and a feed point for feeding the OATs-containing aqueous melamine solution.

**29.** Apparatus according to Claim 27 or 28, **characterized in that** the at least one first vacuum crystallizer has a feed line for a suspension from at least one second crystallization stage.

**30.** Apparatus according to at least one of Claims 27 to 29, **characterized in that** the at least one second vacuum crystallizer has a circuit line having a circulation pump and a feed point for feeding a first OATs- and melamine-containing mother liquor.

**Revendications**

**1.** Procédé de cristallisation en deux étapes de la mélamine, **caractérisé en ce que** une solution aqueuse de mélamine contenant de l'oxo-aminotriazine (OAT)

> a) est soumise à une première étape de cristallisation sous vide à un pH supérieur à 11 et à une température $T_{K1}$, de manière à cristalliser une grande partie de la mélamine, qui est acheminée vers un autre traitement, à obtenir une première solution mère contenant de l'OAT et de la mélamine, ensuite,
> b) au moins une partie de la première solution mère contenant de l'OAT et de la mélamine est soumise à au moins une deuxième étape de cristallisation sous vide à un pH supérieur à 11 et à une température $T_{K2}$, avec $T_{K2} < T_{K1}$, de manière à cristalliser une autre partie de la mélamine, qui est réacheminée vers le procédé de mélamine, et à obtenir une deuxième solution mère contenant de l'OAT, qui est acheminée vers une étape de traitement des eaux usées.

**2.** Procédé selon la revendication 1, **caractérisé en ce qu'**il est couplé à un procédé à haute pression pour la production de mélamine à partir d'urée avec un traitement aqueux.

**3.** Procédé selon la revendication 1 ou 2, **caractérisé en ce que** la solution de mélamine aqueuse contenant l'OAT contient 6 à 15 % en poids de mélamine.

**4.** Procédé selon au moins l'une des revendications précédentes, **caractérisé en ce que** la solution de mélamine aqueuse contenant de l'OAT contient 8 à 12 % en poids de mélamine.

**5.** Procédé selon au moins l'une des revendications précédentes, **caractérisé en ce que** la solution de mélamine aqueuse contenant de l'OAT présente une température de 100 à 200° C.

**6.** Procédé selon au moins l'une des revendications précédentes, **caractérisé en ce que** la solution de mélamine aqueuse contenant de l'OAT présente une température entre 130 et 150° C.

7. Procédé selon au moins l'une des revendications précédentes, **caractérisé en ce que** le pH est ajusté à l'aide de NaOH.

8. Procédé selon au moins l'une des revendications précédentes, **caractérisé en ce que** le pH dans la première et au moins dans une deuxième étape de cristallisation est de 11,8 à 12,5.

9. Procédé selon au moins l'une des revendications précédentes, **caractérisé en ce que** la $T_{K1}$ = 30 à 60° C.

10. Procédé selon au moins l'une des revendications précédentes, **caractérisé en ce que** la $T_{K1}$ = 40 à 50° C.

11. Procédé selon au moins l'une des revendications précédentes, **caractérisé en ce qu'**au moins 20 % en poids, en particulier, 20 à 40 % en poids de la première solution mère contenant de l'OAT et de la mélamine sont soumis à au moins une deuxième étape de cristallisation.

12. Procédé selon au moins l'une des revendications précédentes, **caractérisé en ce qu'**environ 30 % en poids de la première solution mère contenant de l'OAT et de la mélamine sont soumis à au moins une deuxième étape de cristallisation.

13. Procédé selon au moins l'une des revendications précédentes, **caractérisé en ce que** la première solution mère contenant de l'OAT et de la mélamine qui est acheminée vers au moins une deuxième étape de cristallisation, contient la solution mère provenant de la filtration de mélamine.

14. Procédé selon au moins l'une des revendications précédentes, **caractérisé en ce que** la $T_{K2}$ = 15 à 40° C.

15. Procédé selon au moins l'une des revendications précédentes, **caractérisé en ce que** la $T_{K2}$ = 25 à 35° C.

16. Procédé selon au moins l'une des revendications précédentes, **caractérisé en ce que** la première solution mère contenant de l'OAT et de la mélamine ? acheminée vers au moins une deuxième étape de cristallisation, présente une concentration de 1,5 à 2,5 % en poids d'OAT.

17. Procédé selon au moins l'une des revendications précédentes, **caractérisé en ce que** la première solution mère contenant de l'OAT et de la mélamine, acheminée vers au moins une deuxième étape de cristallisation, présente une concentration de 1,9 à 2,2 % en poids d'OAT.

18. Procédé selon au moins l'une des revendications précédentes, **caractérisé en ce que** l'étape de traitement des eaux usées vers laquelle est acheminée la deuxième solution mère contenant de l'OAT, est une installation de traitement des eaux usées thermique.

19. Procédé selon au moins l'une des revendications précédentes, **caractérisé en ce que** la partie de la première solution mère contenant de l'OAT et de la mélamine qui n'est pas acheminée vers au moins une deuxième étape de cristallisation est réacheminée vers la partie de traitement aqueuse de l'installation pour la mélamine.

20. Procédé selon au moins l'une des revendications précédentes, **caractérisé en ce que** la partie de mélamine cristallisée dans au moins une deuxième étape de cristallisation, est recyclée vers le refroidisseur de la première étape de cristallisation ou vers le récipient de mise en suspension après la première étape de cristallisation.

21. Procédé selon au moins l'une des revendications précédentes, **caractérisé en ce que**, à la première étape de cristallisation, le vide est produit au moyen d'un condensateur avec un milieu de refroidissement.

22. Procédé selon au moins l'une des revendications précédentes, **caractérisé en ce que**, dans la première et au moins dans une deuxième étape de cristallisation, le vide est produit au moyen d'un condensateur avec le milieu de refroidissement.

23. Procédé selon au moins l'une des revendications précédentes, **caractérisé en ce que** le vide est produit au moyen d'un condensateur avec milieu de refroidissement, à la première étape de cristallisation, et est produit au moyen d'un éjecteur de vapeur au moins dans une deuxième étape de cristallisation.

**24.** Procédé selon la revendication 23, **caractérisé en ce que**, dans l'éjecteur de vapeur d'au moins une deuxième étape de cristallisation, le rapport de la quantité de la vapeur utilisée à la quantité de matière évaporée se situe entre 1,0 et 1,1.

**25.** Procédé selon au moins l'une des revendications précédentes, **caractérisé en ce que** la condensation des vapeurs de la première et au moins d'une deuxième étape de cristallisation s'effectue dans un condensateur en commun avec le milieu de refroidissement.

**26.** Procédé selon au moins l'une des revendications précédentes, **caractérisé en ce que** l'on fait fonctionner en parallèle deux premières étapes de cristallisation et une deuxième étape de cristallisation.

**27.** Dispositif de réalisation du procédé selon la revendication 1, **caractérisé par** au moins un premier cristallisateur sous vide comportant

    a) un dispositif de production de vide et de condensation de vapeurs,
    b) une conduite pour acheminer la solution de mélamine aqueuse contenant de l'OAT,
    c) une conduite d'évacuation de la mélamine cristallisée,
    d) une conduite de soutirage des vapeurs,
    e) une conduite de soutirage comportant une pompe de soutirage pour une première solution mère contenant de l'OAT et de la mélamine et

au moins un deuxième cristallisateur sous vide comportant

    f) un dispositif de production de vide et de condensation de vapeurs,
    g) une conduite pour l'acheminement de la première solution mère contenant de l'OAT et de la mélamine,
    h) une conduite pour l'évacuation de la mélamine cristallisée
    i) une conduite de soutirage des vapeurs,
    j) une conduite de soutirage comportant une pompe de soutirage pour une deuxième solution mère contenant de l'OAT.

**28.** Dispositif selon la revendication 27, **caractérisé en ce qu'**au moins un premier cristallisoir sous vide présente un circuit comportant une pompe de circulation et un point d'alimentation pour acheminer la solution de mélamine aqueuse contenant de l'OAT.

**29.** Dispositif selon la revendication 27 ou 28, **caractérisé en ce qu'**au moins un premier cristallisateur sous vide présente une conduite d'acheminement d'une suspension provenant d'au moins une deuxième étape de cristallisation.

**30.** Dispositif selon au moins l'une des revendications 27 à 29, **caractérisé en ce qu'**au moins un deuxième cristallisateur sous vide présente un circuit comportant une pompe de circulation et un point d'alimentation pour l'entrée d'une première solution mère contenant de l'OAT et de la mélamine.

Figur 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- IT 01282370
- WO 9937628 A1
- US 3637686 A
- WO 0146159 A2